# EUROPÄISCHE PATENTSCHRIFT

(11) **EP 3 585 245 B1**
(45) Veröffentlichungstag und Bekanntmachung des Hinweises auf die Patenterteilung: **13.04.2022**
(21) Anmeldenummer: 18710773.5
(22) Anmeldetag: 21.02.2018
(51) Int. Cl.: A61B 3/107

(54) **VERFAHREN UND ANORDNUNG ZUR HOCHAUFLÖSENDEN TOPOGRAPHIE DER KORNEA EINES AUGES**
METHOD AND ARRANGEMENT FOR HIGH-RESOLUTION TOPOGRAPHY OF THE CORNEA OF AN EYE
PROCÉDÉ ET DISPOSITIF POUR LA TOPOGRAPHIE À HAUTE RÉSOLUTION DE LA CORNÉE D'UN OEIL

(30) Priorität: 24.02.2017 DE 102017203010
(43) Veröffentlichungstag der Anmeldung: 01.01.2020
(73) Patentinhaber: Carl Zeiss Meditec AG, 07745 Jena (DE)
(72) Erfinder: BUBLITZ, Daniel, 07646 Rausdorf (DE)
(74) Vertreter: Kintzel, Klaus-Peter
(86) Internationale Anmeldenummer: PCT/EP2018/054204
(87) Internationale Veröffentlichungsnummer: WO 2018/153882

(56) Entgegenhaltungen:
- EP-A1- 0 563 454
- DE-A1-102012 011 880

## Beschreibung

Die vorliegende Erfindung betrifft ein Verfahren und eine Anordnung zur Bestimmung der Topografie der Kornea eines Auges, basierend auf einer optischen, berührungslosen Datenerfassung.

Bei der Hornhauttopografie am Auge handelt es sich um eine mikrometergenaue Untersuchungsmethode der Hornhaut, bei der von der Oberfläche der Hornhaut eine Art Landkarte erstellt wird. Der Augenarzt misst das Ausmaß der Krümmung der Hornhaut an tausenden Einzelpunkten. Die Hornhauttopografie am Auge ermöglicht eine exakte Abbildung der Wölbung der Hornhaut. Auf Grundlage dieser Ergebnisse kann der Augenarzt mögliche krankhafte Veränderungen feststellen. Auch für die Korrektur von Refraktionsfehlern ist die exakte Vermessung durch die Hornhauttopografie am Auge von großer Bedeutung.

Im Hinblick auf die neuere Anwendungen, wie beispielsweise:
- die Katarakt-Chirurgie,
- der IOL Linsen Berechnung,
- der Kontaktlinsenanpassung und
- der Refraktiven Laserchirurgie,
kommen auf die Topografieverfahren besondere Herausforderungen hinsichtlich der Genauigkeit und Reproduzierbarkeit der Messungen zu, die es erforderlich machen entweder die traditionellen Topografieverfahren zu verbessern oder neue Verfahren zu entwickeln.

Zu den eher traditionellen Instrumenten zur Messung der Topografie der Hornhaut (lat. Kornea) des Auges zählen neben Keratometern und Scheimpflug-Kameras auch solche, die auf der Projektion eines Placidoring-Systems basieren.

Zu den neueren Topografieverfahren zählt die konfokale, optische Kurzkohärenztomografie (optical coherence tomography, kurz: OCT), die auf der Weißlicht-Interferometrie basiert und die Laufzeit eines Signals mit Hilfe eines Interferometers vergleicht. Dabei wird der Arm eines Interferometers mit bekannter optischer Weglänge (= Referenzarm) als Referenz zum Messarm herangezogen. Die Interferenz der Signale aus beiden Armen (Referenz- und Messarm) ergibt ein Muster, aus dem man die relative optische Weglänge innerhalb eines A-Scans (einzelnes Tiefensignal) herauslesen kann. In den eindimensionalen Rasterverfahren wird der Strahl dann, analog zur Ultraschalltechnik transversal in einer oder zwei Richtungen geführt, womit sich ein flächiger B-Scan oder ein dreidimensionales Tomogramm (C-Scan) aufnehmen lässt. Beispielsweise wird für einen aus 100 einzelnen A-Scans bestehenden B-Scan eine Sekunde Messzeit benötigt.

Die Messauflösung des OCT-Verfahrens wird durch die sogenannte Kohärenzlänge der eingesetzten Lichtquelle bestimmt und liegt typischerweise bei etwa 15µm. Aufgrund seiner besonderen Eignung zur Untersuchung optisch transparenter Medien ist das Verfahren in der Ophthalmologie weit verbreitet.

Bei den in der Ophthalmologie verwendeten OCT-Verfahren haben sich zwei verschiedene Typen durchgesetzt. Zur Bestimmung der Messwerte wird beim ersten Typ der Referenzarm in der Länge verändert und kontinuierlich die Intensität der Interferenz gemessen, ohne dass dabei das Spektrum berücksichtigt wird. Dieses Verfahren wird als "Time Domain"-Verfahren bezeichnet. Bei dem anderen, als "Frequency Domain" bezeichneten Verfahren, wird hingegen zur Bestimmung der Messwerte das Spektrum berücksichtigt und die Interferenz der einzelnen spektralen Komponenten erfasst. Deshalb spricht man einerseits vom Signal in der Zeitdomäne (Time Domain; kurz: TD) und andererseits vom Signal in der Frequenzdomäne (Frequency Domain, kurz FD).

Gegenüber den konventionellen Verfahren der Keratometrie und Placido-Topografie, die mit einer Belichtung des Kamerachips einen Datensatz ohne störende Augenbewegungen erheben können, ist die verhältnismäßig lange Scanzeit der OCT-Verfahren nachteilig und erfordert weitere Korrekturprozeduren.

So beschreibt die EP 0 563 454 A1 eine Lösung zum Untersuchen des Auges mit Hilfe einer Lichtquelle und einem Interferometer. Dabei wird das Auge in einem ersten Strahlengang angeordnet und die von einer Grenzfläche des Auges zurückgeworfene Objektlichtwelle mit einer Referenzlichtwelle zur Interferenz gebracht. Die Interferenzerscheinungen werden detektiert und ausgewertet, wobei erfindungsgemäß vorgesehen ist, dass die Auswertung mittels mindestens eines opto-elektronischen Sensors erfolgt und die Referenzlichtwelle durch definierte optische Elemente zur Interferenz mit der Objektlichtwelle geführt wird. Mit der hier beschriebenen Lösung ist zwar auch die Bestimmung der Topografie der Kornea eines Auges möglich, allerdings kann weder das Vorzeichen der Wellenfrontabweichungen noch der absoluten Krümmungsradius der Kornea bestimmt werden.

Eine weitere Lösung für ein berührungsloses ophthalmologisches Messgerät zur Berechnung und Auswahl einer IOL wird in der DE 10 2012 011 880 A1 beschrieben. Das Messgerät besteht dabei aus einer interferometrischen Anordnung zur Bestimmung der Augenlänge, einer Keratometeranordnung zur Bestimmung der Hornhautkrümmung des Auges, sowie einer Steuer- und Auswerteeinheit zur Erfassung und Auswertung der Daten und zur Auslegung von IOL's. Als eine besonders bevorzugte Variante wird ein Geräteaufbau als handgehaltene Version beschrieben. Dabei wird das kompakte Gerät während der Messung vom Anwender per Hand vor dem Patientenauge positioniert. Eine sehr schnelle Bestimmung aller für die Auswahl einer künstlichen Augenlinse erforderlichen Messwerte ist bei dieser Variante zwingend erforderlich.

Ein Mangel im Stand der Technik ist auch, dass man mit aktuell verfügbaren, kostengünstigen, konfokalen OCT-Systemen noch keine zuverlässigen Topografiedaten der Kornea zur Verfügung stellen kann. Neben der Auflösung und Reproduzierbarkeit ist die lange Scanzeit in Verbindung mit der Augenbewegung eine aktuell nur mit hoch präzisen Eyetrackern prinzipiell beherrschbares Problem.

Kombinationsgeräte mit einer Placidoring-Projektion und einem OCT-Gerät würden einen vergleichsweise große Bauform erfordern, wobei die Placidoscheibe insbesondere die Sicht des Bedieners auf das Patientenaugen versperrt und zu zeitaufwendigeren Abläufen führt. Wenn ein großer Durchmesser bei der Topografie von bis zu ca. 16mm bis in den Bereich der Sklera erreicht werden soll, würde der Placidoprojektor entsprechend größere Dimensionen beanspruchen und das Handling weiterhin erschweren bzw. das optische Design ist sehr eingeschränkt umzusetzen.

Der IOLMaster^{®} 700 von Zeiss [1] erhebt zur Messung der biometrischen axialen Abstände im Auge bereits B-Scans und damit auch ein Datennetz zur Oberflächentopografie der Kornea. Dieses System, bei dem eine visuelle Überprüfung der gewonnenen Biometriedaten anhand von OCT-Bilder stattfindet, zeichnet sich durch bessere Refraktionsergebnisse bei einer hohen Wiederholbarkeit und einer klinischen Datenbank-Anbindung aus. Außerdem ist mit einer vergleichsweise geringen Standardabweichung der Reproduzierbarkeit die Erhebung der OCT-Daten möglich. Allerdings treffen auf dieses System mit einer vergleichsweisen langsamen Scanrate genannter aktueller Nachteile zu.

Ein derartiges Verfahren zur optischen 3D-Bildgebung einer streuenden Probe, insbesondere zur Bestimmung deren räumlichen Streustärkenverteilung wird in der DE 10 2011 018 603 B3 beschrieben. Hierbei wird die Probe mit verschiedenen Wellenlängen beleuchtet, das von der Probe reflektierte Licht sowie das Referenzlicht auf einen 2D-Lichtdetektor-Array abgebildet und für jede der Wellenlängen je ein Hologramm aufgezeichnet. Aus allen berechneten Wellenfeldern wird über verschieden Zwischenschritte Verteilung der Streustärke wenigstens in einer Schicht der Probe rekonstruiert.

Nachteilig bei dieser Lösung der klassischen Holoskopie, die einer Weitfeldanwendung der Swept Source OCT entspricht, ist darin zu sehen, dass sehr viele Bilder zu realisieren und auszuwerten sind, um aus diesem Bilderstapel die Tiefeninformationen zu extrahieren.

### Literatur:

[1] IOLMaster^{®} 700 von Zeiss, DE_32_010_0009II Gedruckt in Deutschland CZ-I/2015; ^{©} Carl Zeiss Meditec AG, 2014.

Der vorliegenden Erfindung liegt die Aufgabe zugrunde eine OCT basierte, biometrische Lösung zu entwickeln, die auch die Bestimmung der Topografie der Kornea eines Auges ermöglicht. Die Lösung soll dabei den wachsenden Anforderungen hinsichtlich Genauigkeit und Reproduzierbarkeit der Messdaten gerecht werden.

Diese Aufgabe wird mit dem vorgeschlagenen Verfahren zur hochauflösenden Topographie der Kornea eines Auges, bei dem das Auge von der Beleuchtungsquelle einer konfokalen, interferometrischen Messanordnung beleuchtet, die von der Kornea reflektierten Lichtsignale sowohl von der konfokalen, interferometrischen Messanordnung, als auch einem ortsauflösenden Detektor erfasst und an eine Auswerteeinheit weitergeleitet werden, dadurch gelöst,
a) dass die Beleuchtungsstrahlung auf den Krümmungsmittelpunkt der Kornea fokussiert wird,
b) dass der von einem Strahlteiler ausgekoppelte und auf den ortsauflösenden Detektor abgebildete Teil des von der Kornea reflektierten Lichtsignals mit einem, von einer Referenzlichtquelle mit einer Delay Line erzeugten Referenzsignal überlagert wird,
c) dass das Referenzsignal eine ähnliche und bekannte Wellenfrontform wie die des von der Kornea reflektierten Lichtsignales aufweist und über die Delay Line so eingestellt wird, das sie zu den von der Kornea reflektierten Lichtsignalen interferenzfähig ist,
d) dass zeitgleich zu dem auf dem ortsauflösenden Detektor erzeugten Interferenzmuster von der interferometrischen Messanordnung der Abstand der Korneaoberfläche von einer Referenzfläche bestimmt wird und
e) dass von der Auswerteeinheit aus den zu mindestens einem Zeitpunkt aufgenommenen Messsignalen des ortsauflösenden Detektors und der interferometrischen Messanordnung die Topographie der Kornea bestimmt wird.

Mit der erfindungsgemäßen Anordnung zur hochauflösenden Topographie der Kornea eines Auges, bestehend aus einer konfokalen, interferometrischen Messanordnung, einem ortsauflösenden Detektor, Vorrichtungen zur Positionierung und einer Auswerteeinheit, wird diese Aufgabe dadurch gelöst, dass die interferometrische Messanordnung auf einem Frequenz-Domain-OCT-Verfahren basiert und dessen Strahlung auf den Krümmungsmittelpunkt der Kornea fokussiert ist, dass ein Strahlteiler zur Auskopplung und Abbildung eines Teiles des von der Kornea reflektierten Lichtsignals auf den ortsauflösenden Detektor vorhanden ist, dass zur Überlagerung des auf den ortsauflösenden Detektor abzubildenden Teiles des von der Kornea reflektierten Lichtsignals mit einem Referenzsignal eine Referenzlichtquelle mit einer Delay Line und einem Strahlteiler vorhanden und vor dem ortsauflösenden Detektor angeordnet sind, dass die Referenzlichtquelle ausgebildet ist ein Referenzsignal mit einer ähnlichen und bekannten Wellenfrontform wie die des von der Kornea reflektierten Lichtsignales zu erzeugen und dass die Auswerteeinheit ausgebildet ist, aus den zeitgleich aufgenommenen Signalen der interferometrischen Messanordnung und des ortsauflösenden Detektors die Topographie der Kornea zu bestimmen.

Während die Aufgabe erfindungsgemäß durch die Merkmale der unabhängigen Ansprüche gelöst wird, sind bevorzugte Weiterbildungen und Ausgestaltungen Gegenstand der abhängigen Ansprüche.

Die vorgeschlagene Lösung dient der Bestimmung der Topographie der Kornea eines Auges und basiert auf einem kohärent optischen Topographiesystem. Erfindungsgemäß kombiniert die Lösung ein konfokales FD-OCT Verfahren mit Elementen der bildgebenden Holoskopie und kann dadurch insbesondere in bereits etablierten Systemen, wie dem IOLMaster^{®} 700 von Zeiss die Topografie der Kornea mit der notwendigen Genauigkeit interferometrisch gemessen werden.

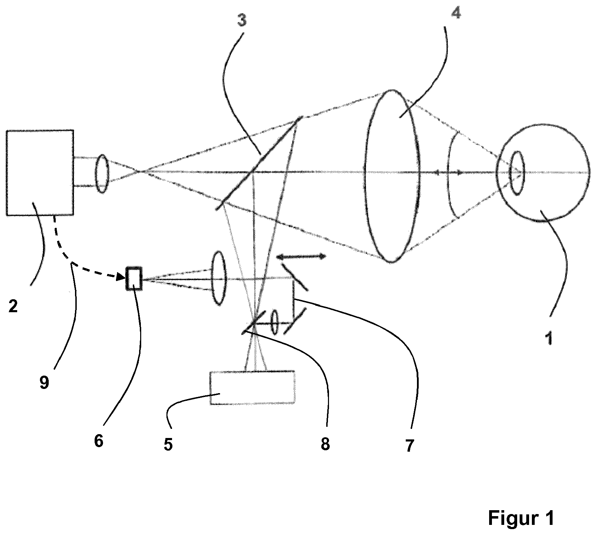

Die Erfindung wird nachfolgend anhand von Ausführungsbeispielen näher beschrieben. Dazu zeigt die
- Figur 1:: eine erfindungsgemäße Anordnung zur hochauflösenden Topographie der Kornea eines Auges.

Bei dem Verfahren zur hochauflösenden Topographie der Kornea eines Auges, wird das Auge von der Beleuchtungsquelle einer konfokalen, interferometrischen Messanordnung beleuchtet, die von der Kornea reflektierten Lichtsignale sowohl von der konfokalen, interferometrischen Messanordnung, als auch einem ortsauflösenden Detektor erfasst und an eine Auswerteeinheit weitergeleitet. Erfindungsgemäß beinhaltet das Verfahren folgende einzelne Verfahrensschritte:
a) dass die Beleuchtungsstrahlung auf den Krümmungsmittelpunkt der Kornea fokussiert wird,
b) dass der von einem Strahlteiler ausgekoppelte und auf den ortsauflösenden Detektor abgebildete Teil des von der Kornea reflektierten Lichtsignals mit einem, von einer Referenzlichtquelle mit einer Delay Line erzeugten Referenzsignal überlagert wird,
c) dass das Referenzsignal eine ähnliche und bekannte Wellenfrontform wie die des von der Kornea reflektierten Lichtsignales aufweist und über die Delay Line so eingestellt wird, das sie zu den von der Kornea reflektierten Lichtsignalen interferenzfähig ist,
d) dass zeitgleich zu dem auf dem ortsauflösenden Detektor erzeugten Interferenzmuster von der interferometrischen Messanordnung der Abstand der Korneaoberfläche von einer Referenzfläche bestimmt wird und
e) dass von der Auswerteeinheit aus den zu mindestens einem Zeitpunkt aufgenommenen Messsignalen des ortsauflösenden Detektors und der interferometrischen Messanordnung die Topographie der Kornea bestimmt wird.

Einer ersten vorteilhaften Ausgestaltung entsprechend verwendet die interferometrische Messanordnung ein Frequenz-Domain-OCT-Verfahren, insbesondere ein Swept-Source OCT-Verfahren. Dabei wird das Auge von der Beleuchtungsquelle der interferometrischen Messanordnung mit einem Beleuchtungsbündel bestrahlt, dessen Öffnungswinkel 20° bis 100°, bevorzugt 60° beträgt.

Die Strahlung der Beleuchtungsquelle wird insbesondere konvergent auf den Krümmungsmittelpunkt der Kornea, der etwa 8mm hinter dem Scheitel einer normalen Kornea liegt fokussiert. Um eine Korneafläche mit einem Durchmesser von etwa 6mm auszuleuchten ist eine Apertur der konvergenten Laserwelle von etwa 45° (Vollwinkel) erforderlich.

Die Messwerterfassung der von der Kornea reflektierten Lichtsignale durch die konfokale, interferometrische Messanordnung erfolgt in einer Ebene, die konjugiert zur Fokusebene der Beleuchtungsstrahlung liegt.

Erfindungsgemäß wird ein Teil der von der Kornea reflektierten Lichtsignale auf den ortsauflösenden Detektor abgebildet.

Von dem dafür vorhandenen Strahlteiler wird ein Teil des von der Kornea reflektierten Lichtsignals in Höhe von 2% bis 50 %, vorzugsweise etwa 10% auskoppelt und auf den ortsauflösenden Detektor abgebildet.

Einer zweiten vorteilhaften Ausgestaltung entsprechend nimmt der ortsauflösende Detektor die Interferenzsignale mit Belichtungszeiten auf, die auf die Durchstimmzeit und den Wellenlängenbereich der Beleuchtungsquelle der interferometrischen Messanordnung abgestimmt sind, so dass effektive Kohärenzlänge im Bereich zwischen 10µm und 1mm realisiert werden.

Erfindungsgemäß liegt die Belichtungszeit des ortsauflösenden Detektors im Bereich von 1% bis 100% der Zeit, die für die Durchstimmung der Beleuchtungsquelle benötigt wird. Diese Belichtungszeit limitiert den messbaren Tiefenbereich. Objektpunkte außerhalb dieses messbaren Tiefenbereichs weisen zeitlich so schnell veränderliche Interferenzerscheinungen auf, dass sich diese aus den Aufnahmen des ortsauflösenden Detektors herausmitteln.

Wie bereits erwähnt, wird der auf den ortsauflösenden Detektor abgebildete Teil des von der Kornea reflektierten Lichtsignals mit einem Referenzsignal überlagert, welches eine ähnliche und bekannte Wellenfrontform wie die des von der Kornea reflektierten Lichtsignales aufweist.

Einer dritten vorteilhaften Ausgestaltung entsprechend wird als Referenzsignal eine Kugelwelle verwendet, deren Zentrum konjugiert zum Krümmungsmittelpunkt der Kornea liegt. Über die Delay Line wird diese so eingestellt, dass sie zu den von der Kornea reflektierten Lichtsignalen interferenzfähig ist. Die Referenzlichtquelle ist erfindungsgemäß aus der durchstimmbaren Beleuchtungsquelle der konfokalen, interferometrischen Messanordnung ausgekoppelt, um mit dieser kohärent zu sein.

Erfindungsgemäß werden in der Nähe des Punktes an dem das Beleuchtungslicht auf den Scheitel der Kornea fokussiert abgebildet wird, mit dem ortsauflösenden Detektor und der interferometrischen Messanordnung zeitgleich mindestens zwei Messsignalpaare mit unterschiedlichem Abstand zum Fokuspunkt aufgenommen, um beide Systeme aufeinander zu kalibrieren. Dazu wird in den mindestens zwei Bildern des ortsaufgelösten Sensors der Durchmesser des Zerstreuungskreises der von der Kornea zurück reflektierten Lichtsignale bestimmt. Trägt man den Durchmesser dieses Zerstreuungskreises über der mit dem konfokalen OCT gemessenen Entfernung zum Scheitel der Kornea auf, so kann man über z. B. eine lineare Regression den Punkt bestimmen an dem das Lichtsignal vollständig fokussiert auf den ortsauflösenden Detektor fallen würde. Da man zu diesem Zustand auch den mit dem konfokalen OCT gemessenen Abstand vom Scheitelpunkt der Kornea kennt, kann man so die beiden Messprinzipien aufeinander kalibrieren und z. B. auf Temperatureffekten basierende Weglängenänderungen in der interferometrischen Messanordnung kompensieren.

Einer weiteren vorteilhaften Ausgestaltung entsprechend wird der Kalibrierungsfaktor dazu verwendet, die ständig von der interferometrischen Messanordnung ermittelten Entfernungsmesssignale für die Einstellung der Delay Line zu nutzen. Vorzugsweise erfolgt die Nachstellung der Delay Line motorisch.

Außerdem lassen sich durch die Kalibrierung beispielsweise thermische Effekte kompensieren, die die Brennweite der Frontlinse oder die Wegdifferenzen im Gesamtaufbau beeinflussen könnten. Dieser Prozess der Kalibrierung wird im Hintergrund durchgeführt und ist für den Nutzer nicht wahrnehmbar. In der Regel erfolgt dies auch erst nach der eigentlichen Messung, um die Messergebnisse entsprechend zu korrigieren.

Durch die stetige Entfernungskorrektur des Referenzsignals wird gewährleistet, dass vom ortsauflösenden Detektor nur noch Aufnahmen realisiert werden, die neben dem Korneareflex auch Interferenzringe beinhalten.

Einer weiteren vorteilhaften Ausgestaltung entsprechend wird in der Nähe des Punktes, an dem das Beleuchtungslicht auf den Krümmungsmittelpunkt der Kornea fokussiert abgebildet wird, mit dem ortsauflösenden Detektor mindestens ein Messsignal aufgenommen, die Abweichungen des von der Kornea reflektierten Lichtsignales von einer idealen Kugelwelle interferometrisch bestimmt und aus dem zeitgleich von der interferometrischen Messanordnung gelieferten Messsignal der absolute Krümmungsradius der Kornea und damit die Topographie der Kornea ermittelt.

Allerdings lässt sich aus nur einem Messsignalpaar das Vorzeichen der Abweichungen des von der Kornea reflektierten Lichtsignales von einer idealen Kugelwelle nicht bestimmen. Dazu wird ein zweites Messsignalpaar benötigt, welches sich im Abstand zum Fokuspunkt leicht unterscheidet. Zwar wird das Delay des auf den ortsauflösenden Detektor abgebildeten Referenzsignals motorisch auf das von der Kornea reflektierte Lichtsignal eingestellt, aber durch zufällige Anteile der Patientenbewegung können geringe und zufällige Abweichungen zum "Zero Delay" auftreten, wodurch der Kontrast der Interferenzringe im Detektorbild variiert.

In den mindestens zwei Messsignalen des ortsauflösenden Detektors wird der Kontrast der Interferenzen am selben Punkt der Kornea verglichen. Das Vorzeichen der Wellenfrontabweichungen kann unter Berücksichtigung der zu den jeweiligen Messsignalpaaren gehörenden, von der konfokalen, interferometrischen Messanordnung ermittelten Abständen zum Scheitel der Kornea eindeutig bestimmt werden.

Einer letzten vorteilhaften Ausgestaltung entsprechend werden in der Nähe des Punktes, an dem das Beleuchtungslicht auf den Krümmungsmittelpunkt der Kornea fokussiert abgebildet wird, mit dem ortsauflösenden Detektor mehrere Messsignale aufgenommen, wobei die Verstellung der Delay Line für das Referenzsignal des ortsauflösenden Detektors deutlich stärker vom Zero-Delay abweicht und dadurch der messbare Tiefenbereich für stark asphärische Krümmungen der Kornea erweitert wird. Für stark asphärisch gekrümmte Korneas kann somit ein resultierendes Messsignale dadurch erreicht werden, dass aus den einzelnen Messungen verfügbare Teile des Messsignales zu einem resultierenden Messsignal zusammengesetzt werden.

Erfindungsgemäß ergibt sich die Genauigkeit für die Bestimmung bei der absoluten Krümmungsradien der Kornea aus den Genauigkeiten der interferometrischen Messung und der kohärent optischen Abstandsmessung. Bei Genauigkeiten der interferometrischen Messung von unter 100nm und von unter 5µm bei der kohärent optischen Abstandsmessung können absolute Krümmungsradien der Kornea mit einer Genauigkeit von ±5µm bestimmt werden.

Das vorgeschlagene Verfahren ist sogar zur Vermessung der Kornea von Augen anwendbar, die z. B. im Falle bestimmter Erkrankungen, wie Keratokonus o. ä. signifikante Abweichungen von einer Kugelform aufweisen.

In den Messsignalen des ortsauflösenden Detektors sind aufgrund eines nicht ausreichenden Tiefenmessbereiches daher nur Interferenzerscheinungen auf einem Teil der zu messenden Korneaoberfläche zu sehen.

In diesem Fall ist es vorteilhaft mehrere Messsignale aufzunehmen, die stärkere Abweichungen vom Fokuspunkt haben. Aus diesen Aufnahmen können überlappende Teile der Topografie der Kornea rekonstruiert und somit aus mehreren derartiger Bilder die Gesamttopographie der Kornea durch wiederholte Rekonstruktion ergänzt und zusammengesetzt werden.

Die vorgeschlagene Anordnung zur hochauflösenden Topographie der Kornea eines Auges besteht aus einer konfokalen, interferometrischen Messanordnung, einer ortsauflösenden Detektor, Vorrichtungen zur Positionierung und einer Auswerteeinheit.

Erfindungsgemäß basiert die interferometrische Messanordnung auf einem Frequenz-Domain-OCT-Verfahren, dessen Strahlung auf den Krümmungsmittelpunkt der Kornea fokussiert ist. Ein vorhandener Strahlteiler dient der Auskopplung und Abbildung eines Teiles des von der Kornea reflektierten Lichtsignals auf den ortsauflösenden Detektor.

Zur Überlagerung des auf den ortsauflösenden Detektor abzubildenden Teiles des von der Kornea reflektierten Lichtsignals mit einem Referenzsignal ist vor dem ortsauflösenden Detektor eine Referenzlichtquelle mit einer Delay Line und einem Strahlteiler angeordnet.

Die Beleuchtungsquelle der interferometrischen Messanordnung ist so ausgelegt, dass die Beleuchtungsstrahlung auf den Krümmungsmittelpunkt der Kornea fokussiert ist und eine Fläche der Kornea mit einem Durchmesser von etwa 6mm ausleuchtet.

Erfindungsgemäß ist die Referenzlichtquelle ausgebildet, ein Referenzsignal mit einer ähnlichen und bekannten Wellenfrontform wie die des von der Kornea reflektierten Lichtsignales zu erzeugen. Die Referenzlichtquelle wird hierbei aus der durchstimmbaren Beleuchtungsquelle der konfokalen, interferometrischen Messanordnung ausgekoppelt, um mit dieser kohärent zu sein.

Die Auswerteeinheit ist ausgebildet, aus den zeitgleich aufgenommenen Signalen der interferometrischen Messanordnung und des ortsauflösenden Detektors die Topographie der Kornea zu bestimmen.

Einer vorteilhaften Ausgestaltung entsprechend basiert die interferometrische Messanordnung auf einem Swept Source OCT-Verfahren, dessen Beleuchtungsquelle ausgebildet ist, ein Beleuchtungsbündel mit einem Öffnungswinkel zwischen 20° und 100°, besonders bevorzugt 60°, zu erzeugen.

Erfindungsgemäß ist der Strahlteiler zur Auskopplung und Abbildung eines Teiles des von der Kornea reflektierten Lichtsignals so ausgebildet, dass der auf den ortsauflösenden Detektor abgebildete Teil zwischen 2% und 50 %, vorzugsweise etwa 10% des gesamten von der Kornea reflektierten Lichtsignals beträgt.

Einer weiteren vorteilhaften Ausgestaltung entsprechend ist die Referenzlichtquelle ausgebildet, zur Überlagerung des auf den ortsauflösenden Detektor abzubildenden Teiles des von der Kornea reflektierten Lichtes eine Kugelwelle zu erzeugen, deren Zentrum konjugiert zum Krümmungsmittelpunkt der Kornea liegt. Außerdem besteht die Delay Line erfindungsgemäß aus einem Faserkollimator und einem Retroreflektor. Vorzugseise erfolgt die Anpassung des Referenzsignals dabei motorisch, wofür der Retroreflektor beispielsweise über einen Tauchspulenantrieb verfügt.

Einer weiteren vorteilhaften Ausgestaltung entsprechend ist die Belichtungszeit des ortsauflösenden Detektors auf die Durchstimmzeit und den Wellenlängenbereich der Beleuchtungsquelle der interferometrischen Messanordnung abgestimmt, so dass eine effektive Kohärenzlänge im Bereich zwischen 10µm und 1mm realisierbar ist.

Hierzu zeigt die **Figur 1** eine erfindungsgemäße Anordnung zur hochauflösenden Topographie der Kornea eines Auges.

Die Anordnung besteht aus einer konfokalen, interferometrischen Messanordnung **2,** die hier nur als Kasten dargestellt ist und die (nicht dargestellte) durchstimmbare Beleuchtungsquelle beinhaltet. Von dieser Beleuchtungsquelle wird die Kornea des Auges **1** über einen ersten Strahlteiler **3** und eine Optik **4** beleuchtet. Die von der Kornea des Auges **1** reflektierten Lichtsignale werden sowohl auf die konfokale, interferometrische Messanordnung **2,** als auch den ortsauflösenden Detektor **5** abgebildet. Die von der Kornea des Auges **1** reflektierten Lichtsignale werden vom ersten Strahlteiler **3** auf die beiden Messanordnungen **2** und **5** aufgeteilt.

Zur Überlagerung des Teiles des von der Kornea des Auges **1** reflektierten Lichtsignales mit einem Referenzsignal sind vor dem ortsauflösenden Detektor **5** ein Referenzlichtquelle **6** mit einer Delay Line **7** und einem zweiten Strahlteiler **8** angeordnet. Die Lichtstrahlung der Referenzlichtquelle **6** wird hierbei über einen Lichtleiter **9** aus der durchstimmbaren Beleuchtungsquelle der konfokalen, interferometrischen Messanordnung **2** ausgekoppelt, um mit dieser kohärent zu sein. Die (nicht dargestellte) Auswerteeinheit ist ausgebildet, aus den zeitgleich aufgenommenen Signalen der interferometrischen Messanordnung **2** und des ortsauflösenden Detektors **5** die Topographie der Kornea des Auges **1** zu bestimmen.

Mit der erfindungsgemäßen Lösung werden ein Verfahren und eine Anordnung zur hochauflösenden Topographie der Kornea eines Auges zur Verfügung gestellt, die auf einer optischen, berührungslosen Datenerfassung basiert.

Die vorliegende, OCT basierte Lösung ermöglicht sowohl die Erfassung biometrischer als auch topografischer Werte eines Auges und wird dabei den wachsenden Anforderungen hinsichtlich Genauigkeit und Reproduzierbarkeit der Messdaten gerecht.

Mit einer Interferometrie-basierten Topographiemessung kann eine hohe Genauigkeit der Messwerte nur erreicht werden, wenn der Abstand zum Auge zusätzlich bekannt ist. Im Gegensatz dazu ist bei einer konfokalen OCT-basierten Topographiemessung zwar der Abstand zum Auge bekannt, allerdings wird durch die sequentielle Oberflächenabtastung mit der geringeren Auflösung des OCT-Systems nur eine unzureichende Genauigkeit der Messwerte erreicht. Die vorgeschlagene Lösung kombiniert die positiven Eigenschaften beider Arten der Topographiemessung.

Von Vorteil bei der vorgeschlagenen Lösung ist, dass eine Beleuchtungsquelle die Messstrahlung sowohl für die konfokalen, interferometrische als auch die ortsauflösende Kamera-Messanordnung liefert. Dadurch bleibt die Anzahl der zusätzlich benötigten optischen Bauelemente gering. Auf aufwendige Beleuchtungsanordnungen zur Bestimmung der Topographie kann ebenso verzichtet werden wie eine telezentrisch korrigierte Detektionsoptik.

## Patentansprüche

1. Verfahren zur hochauflösenden Topographie der Kornea eines Auges, bei dem das Auge von der Beleuchtungsquelle einer konfokalen, interferometrischen Messanordnung beleuchtet, die von der Kornea reflektierten Lichtsignale sowohl von der konfokalen, interferometrischen Messanordnung, als auch einem ortsauflösenden Detektor erfasst und an eine Auswerteeinheit weitergeleitet werden, wobei
a) die Beleuchtungsstrahlung auf den Krümmungsmittelpunkt der Kornea fokussiert wird,
b) der von einem Strahlteiler ausgekoppelte und auf den ortsauflösenden Detektor abgebildete Teil des von der Kornea reflektierten Lichtsignals mit einem, von einer Referenzlichtquelle mit einer Delay Line erzeugten Referenzsignal überlagert wird,
c) das Referenzsignal eine ähnliche und bekannte Wellenfrontform wie die des von der Kornea reflektierten Lichtsignales aufweist und über die Delay Line so eingestellt wird, das sie zu den von der Kornea reflektierten Lichtsignalen interferenzfähig ist,
d) zeitgleich zu dem auf dem ortsauflösenden Detektor erzeugten Interferenzmuster von der interferometrischen Messanordnung der Abstand der Korneaoberfläche von einer Referenzfläche bestimmt wird und
e) von der Auswerteeinheit aus den zu mindestens einem Zeitpunkt aufgenommenen Messwerten des ortsauflösenden Detektors und der interferometrischen Messanordnung die Topographie der Kornea bestimmt wird.

2. Verfahren nach Anspruch 1, **dadurch gekennzeichnet, dass** die interferometrische Messanordnung ein Frequenz-Domain-OCT-Verfahren, insbesondere ein Swept-Source OCT-Verfahren verwendet.

3. Verfahren nach Anspruch 1, **dadurch gekennzeichnet, dass** die Beleuchtungsquelle das Auge mit einem Beleuchtungsbündel beleuchtet, dessen Öffnungswinkel 20° bis 100°, bevorzugt 60° beträgt.

4. Verfahren nach Anspruch 1, **dadurch gekennzeichnet, dass** der Strahlteiler zur Abbildung auf den ortsauflösenden Detektor einen Teil des von der Kornea reflektierten Lichtsignals in Höhe von 2% bis 50 %, vorzugsweise etwa 10% auskoppelt.

5. Verfahren nach den Ansprüchen 1 und 4, **dadurch gekennzeichnet, dass** der ortsauflösenden Detektors die Interferenzsignale mit Belichtungszeiten aufnimmt, die auf die Durchstimmzeit und den Wellenlängenbereich der Beleuchtungsquelle der interferometrischen Messanordnung abgestimmt sind, so dass effektive Kohärenzlänge im Bereich zwischen 10µm und 1mm realisiert werden.

6. Verfahren nach Anspruch 1, **dadurch gekennzeichnet, dass** als Referenzsignal eine Kugelwelle verwendet wird, deren Zentrum konjugiert zum Krümmungsmittelpunkt der Kornea liegt.

7. Verfahren nach den Ansprüchen 1 und 6, **dadurch gekennzeichnet, dass** in der Nähe des Punktes an dem das Beleuchtungslicht auf den Scheitel der Kornea fokussiert abgebildet wird, mit dem ortsauflösenden Detektor und der interferometrischen Messanordnung zeitgleich mindestens zwei Messsignalpaare mit unterschiedlichem Abstand zum Fokuspunkt aufgenommen werden, um beide Systeme aufeinander zu kalibrieren.

8. Verfahren nach den Ansprüchen 1, 6 und 7, **dadurch gekennzeichnet, dass** die interferometrische Messanordnung ständig Entfernungsmesssignale liefert, die zur Einstellung der Delay Line genutzt werden.

9. Verfahren nach Anspruch 1, **dadurch gekennzeichnet, dass** in der Nähe des Punktes, an dem das Beleuchtungslicht auf den Krümmungsmittelpunkt der Kornea fokussiert abgebildet wird, mit dem ortsauflösenden Detektor mindestens ein Messsignal aufgenommen, die Abweichungen des von der Kornea reflektierten Lichtsignales von einer idealen Kugelwelle interferometrisch bestimmt und aus dem zeitgleich von der interferometrischen Messanordnung gelieferten Messsignal der absolute Krümmungsradius der Kornea und damit die Topographie der Kornea ermittelt wird.

10. Verfahren nach Anspruch 1, **dadurch gekennzeichnet, dass** in der Nähe des Punktes, an dem das Beleuchtungslicht auf den Krümmungsmittelpunkt der Kornea fokussiert abgebildet wird, mit dem ortsauflösenden Detektor mehrere Messsignale aufgenommen werden, dabei die Verstellung der Delay Line für das Referenzsignal des ortsauflösenden Detektors deutlich stärker vom Zero-Delay abweicht, um für stark asphärische Krümmungen der Kornea den messbaren Tiefenbereich zu erweitern, wobei die Messsignale aus den einzelnen Teilmesssignalen zusammengesetzt und/oder interpoliert werden.

11. Anordnung zur hochauflösenden Topographie der Kornea eines Auges, bestehend aus einer konfokalen, interferometrischen Messanordnung, einem ortsauflösenden Detektor, Vorrichtungen zur Positionierung und einer Auswerteeinheit, wobei die interferometrische Messanordnung (2) auf einem Frequenz-Domain-OCT-Verfahren basiert und dessen Strahlung auf den Krümmungsmittelpunkt der Kornea des Auges (1) fokussiert ist; ein erster Strahlteiler (3) zur Auskopplung und Abbildung eines Teiles des von der Kornea des Auges (1) reflektierten Lichtsignals auf den ortsauflösenden Detektor (5) vorhanden ist; zur Überlagerung des auf den ortsauflösenden Detektor (5) abzubildenden Teiles des von der Kornea des Auges (1) reflektierten Lichtsignals mit einem Referenzsignal eine Referenzlichtquelle (6) mit einer Delay Line (7) und einem zweiten Strahlteiler (8) vorhanden und vor dem ortsauflösenden Detektor (5) angeordnet sind; die Referenzlichtquelle (6) ausgebildet ist ein Referenzsignal mit einer ähnlichen und bekannten Wellenfrontform wie die des von der Kornea des Auges (1) reflektierten Lichtsignales zu erzeugen ; und die Auswerteeinheit ausgebildet ist, aus den zeitgleich aufgenommenen Signalen der interferometrischen Messanordnung (2) und des ortsauflösenden Detektors (5) die Topographie der Kornea des Auges (1) zu bestimmen.

12. Anordnung nach Anspruch 11, **dadurch gekennzeichnet, dass** die interferometrische Messanordnung (2) auf einem Swept Source OCT-Verfahren basiert.

13. Anordnung nach Anspruch 11, **dadurch gekennzeichnet, dass** die Beleuchtungsquelle der interferometrischen Messanordnung (2) ausgebildet ist ein Beleuchtungsbündel mit einem Öffnungswinkel zwischen 20° und 100°, besonders bevorzugt 60°, zu erzeugen.

14. Anordnung nach Anspruch 11, **dadurch gekennzeichnet, dass** der erste Strahlteiler (3) zur Auskopplung und Abbildung eines Teiles des von der Kornea des Auges (1) reflektierten Lichtsignals so ausgebildet ist, dass der auf den ortsauflösenden Detektor (5) abgebildete Teil zwischen 2% und 50 %, vorzugsweise etwa 10% beträgt.

15. Anordnung nach Anspruch 11, **dadurch gekennzeichnet, dass** die Referenzlichtquelle (6) ausgebildet ist zur Überlagerung des auf den ortsauflösenden Detektor (5) abzubildenden Teiles des von der Kornea des Auges (1) reflektierten Lichtes eine Kugelwelle zu erzeugen, deren Zentrum konjugiert zum Krümmungsmittelpunkt der Kornea des Auges (1) liegt.

16. Anordnung nach Anspruch 11, **dadurch gekennzeichnet, dass** die Delay Line (7) aus einem Faserkollimator und einem Retroreflektor besteht.

17. Anordnung nach Anspruch 11, **dadurch gekennzeichnet, dass** die Belichtungszeit des ortsauflösenden Detektors (5) auf die Durchstimmzeit und den Wellenlängenbereich der Beleuchtungsquelle der interferometrischen Messanordnung (2) abgestimmt ist, so dass eine effektive Kohärenzlänge im Bereich zwischen 10µm und 1mm realisierbar ist.

## Claims

1. Method for high-resolution topography of the cornea of an eye,
in which the eye is illuminated by the illumination source of a confocal, interferometric measuring arrangement, the light signals reflected by the cornea are detected both by the confocal, interferometric measuring arrangement and by a spatially resolving detector and said light signals are forwarded to an evaluation unit, wherein
a) the illumination radiation is focused on the centre of curvature of the cornea,
b) the part of the light signal reflected by the cornea that is output coupled from a beam splitter and imaged on the spatially resolving detector is superposed by a reference signal that is produced by a reference light source with a delay line,
c) the reference signal has a similar and known wavefront form like that of the light signal reflected by the cornea and said reference signal is set by way of the delay line in such a way that it is able to interfere with the light signals reflected by the cornea,
d) the distance of the corneal surface from a reference face is determined by the interferometric measuring arrangement at the same time as the interference pattern is produced on the spatially resolving detector and
e) the topography of the cornea is determined by the evaluation unit from the measurement values of the spatially resolving detector and the interferometric measuring arrangement recorded at at least one time.

2. Method according to Claim 1, **characterized in that** the interferometric measuring arrangement uses a frequency domain OCT method, in particular a swept source OCT method.

3. Method according to Claim 1, **characterized in that** the illumination source illuminates the eye with an illumination beam whose aperture angle is 20° to 100°, preferably 60°.

4. Method according to Claim 1, **characterized in that** the beam splitter for imaging on the spatially resolving detector output couples part of the light signal reflected by the cornea, said part corresponding to 2% to 50%, preferably approximately 10%.

5. Method according to Claims 1 and 4, **characterized in that** the spatially resolving detector records the interference signals with exposure times that are matched to the tuning time and the wavelength range of the illumination source of the interferometric measuring arrangement such that effective coherence lengths in the range of between 10 µm and 1 mm are realized.

6. Method according to Claim 1, **characterized in that** a spherical wave is used as a reference signal, the centre of said spherical wave lying conjugate to the centre of curvature of the cornea.

7. Method according to Claims 1 and 6, **characterized in that**, in the vicinity of the point at which the illumination light is imaged in focus on the apex of the cornea, at least two measurement signal pairs with different distances from the focal point are recorded simultaneously by the spatially resolving detector and the interferometric measuring arrangement for the purposes of calibrating both systems to one another.

8. Method according to Claims 1, 6 and 7, **characterized in that** the interferometric measuring arrangement continuously supplies distance measurement signals, which are used to set the delay line.

9. Method according to Claim 1, **characterized in that** at least one measurement signal is recorded by the spatially resolving detector in the vicinity of the point at which the illumination light is imaged in focus on the centre of curvature of the cornea, the deviations of the light signal reflected by the cornea from an ideal spherical wave are determined by interferometry and the absolute radius of curvature of the cornea, and hence the topography of the cornea, is established from the measurement signal supplied by the interferometric measuring arrangement at the same time.

10. Method according to Claim 1, **characterized in that** a plurality of measurement signals are recorded by the spatially resolving detector in the vicinity of the point at which the illumination light is imaged in focus on the centre of curvature of the cornea, the adjustment of the delay line for the reference signal of the spatially resolving detector deviates significantly more strongly from the zero delay in the process in order to expand the measurable depth range for strongly aspherical curvatures of the cornea, wherein the measurement signals are assembled and/or interpolated from the individual partial measurement signals.

11. Arrangement for high-resolution topography of the cornea of an eye, consisting of a confocal, interferometric measuring arrangement, a spatially resolving detector, apparatuses for positioning purposes and an evaluation unit, wherein the interferometric measuring arrangement (2) is based on a frequency domain OCT method and the radiation thereof is focused on the centre of curvature of the cornea of the eye (1); a first beam splitter (3) is present for output coupling and imaging onto the spatially resolving detector (5) some of the light signal reflected by the cornea of the eye (1); a reference light source (6) with a delay line (7) and a second beam splitter (8) is present for superposing the part of the light signal reflected by the cornea of the eye (1) that is to be imaged onto the spatially resolving detector (5) with a reference signal and said reference light source is arranged upstream of the spatially resolving detector (8); the reference light source (6) is embodied to produce a reference signal with a similar and known wavefront form like that of the light signal reflected by the cornea of the eye (1); and the evaluation unit is embodied to determine the topography of the cornea of the eye (1) from the simultaneously recorded signals of the interferometric measuring arrangement (2) and of the spatially resolving detector (5) .

12. Arrangement according to Claim 11, **characterized in that** the interferometric measuring arrangement (2) is based on a swept source OCT method.

13. Arrangement according to Claim 11, **characterized in that** the illumination source of the interferometric measuring arrangement (2) is embodied to produce an illumination beam with an aperture angle of between 20° and 100°, particularly preferably 60°.

14. Arrangement according to Claim 11, **characterized in that** the first beam splitter (3) for output coupling and imaging some of the light signal reflected by the cornea of the eye (1) is embodied in such a way that the part imaged on the spatially resolving detector (5) is between 2% and 50%, preferably approximately 10%.

15. Arrangement according to Claim 11, **characterized in that** the reference light source (6) is embodied to produce a spherical wave for the purposes of superposing the part of the light reflected by the cornea of the eye (1) that is to be imaged onto the spatially resolving detector (5), the centre of said spherical wave lying conjugate to the centre of curvature of the cornea of the eye (1).

16. Arrangement according to Claim 11, **characterized in that** the delay line (7) consists of a fibre collimator and a retroreflector.

17. Arrangement according to Claim 11, **characterized in that** the exposure time of the spatially resolving detector (5) is matched to the tuning time and the wavelength range of the illumination source of the interferometric measuring arrangement (2) such that an effective coherence length in the range of between 10 µm and 1 mm is realizable.

## Revendications

1. Procédé de topographie à haute résolution de la cornée d'un œil, avec lequel l'œil est éclairé par la source d'éclairage d'un arrangement de mesure interférométrique confocal, les signaux lumineux réfléchis par la cornée sont détectés à la fois par l'arrangement de mesure interférométrique confocal et par un détecteur à résolution spatiale et sont retransmis à une unité d'interprétation,
a) le rayonnement d'éclairage étant focalisé sur le point central de la courbure de la cornée,
b) la partie du signal lumineux réfléchi par la cornée qui est découplée par un séparateur de faisceaux et représentée sur le détecteur à résolution spatiale est superposée à un signal de référence généré par une source de lumière de référence comprenant une ligne à retard,
c) le signal de référence possède une forme de front d'onde connue et similaire à celle du signal lumineux réfléchi par la cornée et qui est réglée par le biais de la ligne à retard de telle sorte que les signaux lumineux réfléchis par la cornée sont capables d'interférence,
d) la distance entre la surface de la cornée et une surface de référence est déterminée par l'arrangement de mesure interférométrique simultanément avec le modèle d'interférence généré sur le détecteur à résolution spatiale et
e) la topographie de la cornée est déterminée par l'unité d'interprétation à partir des valeurs mesurées du détecteur à résolution spatiale et de l'arrangement de mesure interférométrique enregistrées à au moins un instant.

2. Procédé selon la revendication 1, **caractérisé en ce que** l'arrangement de mesure interférométrique emploie un procédé OCT dans le domaine fréquentiel, notamment un procédé OCT à source à balayage de fréquence.

3. Procédé selon la revendication 1, **caractérisé en ce que** la source d'éclairage éclaire l'œil avec un faisceau d'éclairage dont l'angle d'ouverture est de 20° à 100°, de préférence de 60°.

4. Procédé selon la revendication 1, **caractérisé en ce que** le séparateur de faisceaux, en vue de la représentation sur le détecteur à résolution spatiale, découple une partie du signal lumineux réfléchi par la cornée à hauteur de 2 % à 50 %, de préférence d'environ 10 %.

5. Procédé selon les revendications 1 et 4, **caractérisé en ce que** le détecteur à résolution spatiale enregistre les signaux d'interférence avec des temps d'exposition qui sont accordés sur le temps d'ajustement et la plage de longueurs d'onde de la source d'éclairage de l'arrangement de mesure interférométrique, de sorte que des longueurs de cohérence effectives dans la plage entre 10 µm et 1 mm sont réalisées.

6. Procédé selon la revendication 1, **caractérisé en ce que** le signal de référence utilisé est une onde sphérique dont le centre est conjugué avec le point central de la courbure de la cornée.

7. Procédé selon les revendications 1 et 6, **caractérisé en ce qu'**au moins deux paires de signaux de mesure se trouvant à des écarts différents du point focal sont enregistrées avec le détecteur à résolution spatiale et l'arrangement de mesure interférométrique à proximité du point auquel la lumière d'éclairage est représentée focalisée sur le sommet de la cornée afin de calibrer les deux systèmes l'un sur l'autre.

8. Procédé selon les revendications 1, 6 et 7, **caractérisé en ce que** l'arrangement de mesure interférométrique délivre continuellement des signaux de mesure de distance qui sont utilisés pour le réglage de la ligne à retard.

9. Procédé selon la revendication 1, **caractérisé en ce qu'**à proximité du point auquel la lumière d'éclairage est représentée focalisée sur le point central de la courbure de la cornée, au moins un signal de mesure est enregistré avec le détecteur à résolution spatiale, les écarts du signal lumineux réfléchi par la cornée par rapport à une onde sphérique idéale sont déterminés de manière interférométrique et le rayon de courbure absolu de la cornée, et ainsi la topographie de la cornée, sont déterminés à partir du signal de mesure délivré simultanément par l'arrangement de mesure interférométrique.

10. Procédé selon la revendication 1, **caractérisé en ce qu'**à proximité du point auquel la lumière d'éclairage est représentée focalisée sur le point central de la courbure de la cornée, plusieurs signaux de mesure sont enregistrés avec le détecteur à résolution spatiale, le positionnement de la ligne à retard pour le signal de référence du détecteur à résolution spatiale s'écartant ici nettement plus fortement du retard nul afin d'élargir la plage de profondeurs mesurable pour des courbures fortement asphériques de la cornée, les signaux de mesure étant composés et/ou interpolés à partir des signaux de mesure partiels individuels.

11. Arrangement de topographie à haute résolution de la cornée d'un œil, constitué d'un arrangement de mesure interférométrique confocal, d'un détecteur à résolution spatiale, de dispositifs de positionnement et d'une unité d'interprétation,
l'arrangement de mesure interférométrique (2) se basant sur un procédé OCT dans le domaine fréquentiel et sont rayonnement étant focalisé sur le point central de la courbure de la cornée de l'œil (1) ;
un premier séparateur de faisceaux (3) destiné à découpler et à représenter une partie du signal lumineux réfléchi par la cornée de l'œil (1) sur le détecteur à résolution spatiale (5) étant présent ;
une source de lumière de référence (6) comprenant une ligne à retard (7) et un deuxième séparateur de faisceaux (8) étant présents et disposés avant le détecteur à résolution spatiale (5) afin de superposer la partie du signal lumineux réfléchi par la cornée de l'œil (1) à représenter sur le détecteur à résolution spatiale (5) avec un signal de référence ;
la source de lumière de référence (6)est configurée pour générer un signal de référence ayant une forme de front d'onde connue et similaire à celle du signal lumineux réfléchi par la cornée de l'œil (1) ; et
l'unité d'interprétation est configurée pour déterminer la topographie de la cornée de l'œil (1) à partir des signaux enregistrés simultanément de l'arrangement de mesure interférométrique (2) et du détecteur à résolution spatiale (5).

12. Arrangement selon la revendication 11, **caractérisé en ce que** l'arrangement de mesure interférométrique (2) se base sur un procédé OCT à source à balayage de fréquence.

13. Arrangement selon la revendication 11, **caractérisé en ce que** la source d'éclairage de l'arrangement de mesure interférométrique (2) est configurée pour générer un faisceau d'éclairage ayant un angle d'ouverture entre 20° et 100°, notamment de préférence égal à 60°.

14. Arrangement selon la revendication 11, **caractérisé en ce que** le premier séparateur de faisceaux (3), en vue du découplage et de la représentation d'une partie du signal lumineux réfléchi par la cornée de l'œil (1), est configuré de telle sorte que la partie du signal lumineux représentée sur le détecteur à résolution spatiale (5) est comprise entre 2 % et 50 %, de préférence égale à environ 10 %.

15. Arrangement selon la revendication 11, **caractérisé en ce que** la source de lumière de référence (6) est configurée pour, en vue de la superposition de la partie de la lumière réfléchie par la cornée de l'œil (1) à représenter sur le détecteur à résolution spatiale (5), générer une onde sphérique dont le centre est conjugué avec le point central de la courbure de la cornée de l'œil (1).

16. Arrangement selon la revendication 11, **caractérisé en ce que** la ligne à retard (7) se compose d'un collimateur à fibres et d'un rétroréflecteur.

17. Arrangement selon la revendication 11, **caractérisé en ce que** le temps d'exposition du détecteur à résolution spatiale (5) est accordé sur le temps d'ajustement et la plage de longueurs d'onde de la source d'éclairage de l'arrangement de mesure interférométrique (2), de sorte qu'une longueur de cohérence effective dans la plage entre 10 µm et 1 mm soit réalisable.
